(19) Europäisches Patentamt
European Patent Office
Office européen des brevets

(11) EP 2 140 854 A1

(12) EUROPEAN PATENT APPLICATION

(43) Date of publication:
06.01.2010 Bulletin 2010/01

(51) Int Cl.:
*A61K 8/29* (2006.01) *A61Q 1/00* (2006.01)

(21) Application number: 08159449.1

(22) Date of filing: 01.07.2008

(84) Designated Contracting States:
AT BE BG CH CY CZ DE DK EE ES FI FR GB GR HR HU IE IS IT LI LT LU LV MC MT NL NO PL PT RO SE SI SK TR
Designated Extension States:
AL BA MK RS

(71) Applicant: The Procter & Gamble Company
Cincinnati OH 45202 (US)

(72) Inventors:
- Elliott, Russell, Phillip
Barnard Castle, Durham DL12 9PN (GB)
- Watson, Joanne
Ossett, Yorkshire WF5 0TJ (GB)

(74) Representative: Wilding, Richard Alan
Procter & Gamble
Technical Centre Ltd
Rusham Park
Whitehall Lane
Egham, Surrey TW20 9NW (GB)

# (54) Cosmetic Composition

(57) Cosmetic composition providing a high coverage of skin while retaining a natural skin appearance comprising iron oxide particles having an average surface area from 30 $m^2/g$ to 150 $m^2/g$, iron-containing titanium dioxide particles having an average surface area from 1 $m^2/g$ to 30 $m^2/g$ and comprising from 1% to 15% iron by weight of the titanium dioxide, and a cosmetically acceptable carrier and its use as a foundation and/or as a composition to correct skin discoloration surrounding the eye.

EP 2 140 854 A1

## Description

### FIELD OF THE INVENTION

According to a first aspect, the present invention relates to a cosmetic composition comprising transparent iron oxide particles, iron-containing titanium dioxide particles, and a cosmetically acceptable carrier. According to a second aspect, the invention relates to the use of this composition as a foundation. According to a third aspect, the invention relates to the use of this composition to correct dark circles around the eyes. Cosmetic compositions according to the present invention may be used to cover skin imperfections, while, at the same time, retaining a natural skin appearance.

### BACKGROUND OF THE INVENTION

Cosmetic compositions are used by consumers for regulating the condition of the skin and/or for improving the appearance of the skin. Cosmetic compositions, such as foundations, are popular amongst consumers, because they are capable of masking skin imperfections and skin tonal variations - this ability is referred to as "coverage". These compositions may also provide coloration to the skin by incorporating conventional metal oxide pigments into the compositions, such as iron oxide pigments and titanium dioxide pigments. Cosmetic compositions, such as eye contour treating compositions, are also popular amongst consumers, because they are capable of correcting skin discoloration surrounding the eyes.

Depending on the consumers' taste and/or the degree of skin imperfections and/or the skin tonal variations and/or the skin discoloration surrounding the eyes, a high coverage effect may be desired and/or needed. A high coverage effect may be obtained by incorporating a high proportion of pigments, especially pigmentary grade titanium dioxide particles, into the cosmetic compositions. However, the application onto the skin of a composition providing high coverage usually impairs the natural appearance of the skin. In particular, the application onto the skin of a composition providing high coverage usually results in a white/blue hue across the skin. The white/blue hue is also known as "ashiness" for consumers having darker skins and "pastiness" for consumers having lighter skins. There is a need, therefore, to provide a cosmetic composition providing high coverage, particularly for masking skin imperfections and/or tonal variations of the skin, while retaining a natural skin appearance. There is also a need to provide a cosmetic composition correcting the skin discoloration surrounding the eyes, while retaining a natural skin appearance. There is also a need to provide a cosmetic composition which delivers high coverage whilst imparting a minimal white/blue hue (ghost-like effect) to the skin, or even whilst imparting no white/blue hue to the skin. There is also a need to provide a cosmetic composition which delivers high coverage to darker skin whilst imparting minimal ashiness, or even whilst imparting no ashiness. There is also a need to provide a cosmetic composition which delivers high coverage to lighter skin whilst imparting minimal pastiness, or even whilst imparting no pastiness. Finally, there is a need to provide a cosmetic composition which delivers high coverage to the skin which minimizes further the white/blue hue to the skin, compare to currently marketed cosmetic compositions.

Ashiness and/or pastiness, i.e. the white/blue hue across the skin, and more generally an unnatural appearance of the skin, is generally associated with the reflectance of violet & blue light. This effect is particularly noticeable when measured at the 110° angle on the X-Rite MA68 II, 5 angle spectrophotomter (called specifically blue & violet light backscatter). When using conventional titanium dioxide, there is a significant reflectance observed in the violet & blue regions (electromagnetic rays having wavelengths from 380 to 495 nm) of the visible light spectrum. This undesirable effect may be reduced by incorporating a lower proportion of pigmentary grade titanium dioxide particles into the cosmetic compositions, but doing that has the disadvantage of reducing the coverage provided to the skin. There is a need, therefore, to provide a cosmetic composition which provides high coverage of skin while minimizing the reflectance of violet & blue light.

### SUMMARY OF THE INVENTION

According to a first aspect, the present invention relates to a cosmetic composition comprising iron oxide particles having an average surface area from 30 $m^2/g$ to 150 $m^2/g$; iron-containing titanium dioxide particles having an average surface area from 1 $m^2/g$ to 30 $m^2/g$ and comprising from 1% to 15% iron by weight of titanium dioxide; and, a cosmetically acceptable carrier. This composition is suitable for providing high coverage while minimizing the reflectance of violet & blue light.

According to a second aspect, the present invention further relates to the use of the cosmetic compositions as foundations.

According to a third aspect, the present invention further relates to the use of the cosmetic compositions to correct dark circles around the eye.

Definitions

As used herein, the term "cosmetic composition" means a composition which is intended to be applied onto the consumer's skin, particularly onto the facial skin or onto the facial skin area surrounding the eyes, so as to regulate the condition of the skin and/or to improve the appearance of the skin.
The term "foundation" means a cosmetic composition which is intended to be applied onto the consumer's skin, particularly, onto the facial skin, so as to provide coverage and/or to mask skin irregularities and/or skin imperfections and/or skin tonal variations.
The term "eye contour treating composition" means a composition which is intended to be applied onto the consumer's skin area surrounding the eye, so as to correct the skin discoloration.
The term "lighter skin" means skin whose mean lightness L* is at least 60, wherein the L* value is in the CIE 1976 colorometric space, using D65 illumination with a 10° observer angle (D65/10 reported). The mean lightness L* may be measured with an apparatus such as a d/8 Portable Integrating Sphere Spectrophotometer supplied by Datacolor (Datacolor International, WA14 5UA, United Kingdom).
The term "darker skin" means skin whose mean lightness L* is less than 60, wherein the L* value is in the CIE 1976 colorometric space, using D65 illumination with a 10° observer angle (D65/10 reported). The mean lightness L* may be measured with an apparatus such as a d/8 Portable Integrating Sphere Spectrophotometer supplied by Datacolor (Datacolor International, WA14 5UA, United Kingdom).
The term "ashiness" means the white/blue hue which is observed onto skin after applying onto skin, particularly darker skin, a cosmetic composition providing high coverage.
The term "pastiness" means the white/blue hue which is observed onto skin after applying onto skin, particularly lighter skin, a cosmetic composition providing high coverage.
The term "violet & blue light back-scatter" means the light reflected between 380 nm and 495 nm, more preferably between 420 nm and 450 nm, at the 110° angle measured using an X-Rite™ MA68 II Spectrophotometer.
The term "transparent iron oxide particles" refers to iron oxide particles having an average surface area of from 30 $m^2/g$ to 150 $m^2/g$, preferably iron oxide particles having an average surface area of from 30 $m^2/g$ to 150 $m^2/g$ and an average primary particle size of less than 100 nm.
The term "average primary particle size" of iron-containing titanium dioxide particles means the equivalent volume mean primary particle size of the elementary iron-containing titanium dioxide crystals, wherein the equivalent volume mean is also known as D[4,3]. The average primary particle size is measured on the iron-containing titanium dioxide particles, before being coated.
The term "average primary particle size" of the iron oxide particles having an average surface area of from 30 $m^2/g$ to 150 $m^2/g$ means the number-length mean primary particle size of the elementary iron oxide crystals, wherein the number-length mean is also known as D[1,0]. The average primary particle size is measured on these iron oxide particles, before being coated.
The term "average surface area" means the average surface area of particles in square meters per gram established by the nitrogen adsorption using the BET (Brunauer-Emmett-Teller) equation. This method consists in the measure, over a range of partial pressures, of the volume of gas ($N_2$) being adsorbed at the surface of the particles. The surface area is proportional to the volume of gas adsorbed. An appropriate device for carrying out the method is the ASAP 2020N apparatus provided by Micromeritics.
The terms "weight percentage" and "percentage by weight" of a component mean the percentage of the active component and not the percentage of the raw material comprising the actives, unless otherwise specified (see examples).
All percentages, ratios and proportions herein are by weight, unless otherwise specified. All such weights as they pertain to listed ingredients are based on the active level, unless otherwise specified.

DETAILED DESCRIPTION OF THE INVENTION

The cosmetic composition, according to the present invention, comprises iron oxide particles having an average surface area from 30 $m^2/g$ to 150 $m^2/g$, iron-containing titanium dioxide particles having an average surface area from 1 $m^2/g$ to 30 $m^2/g$ and comprising from 1 % to 15% iron by weight of titanium dioxide; and, a cosmetically acceptable carrier.
The inventors have surprisingly found that this composition may provide coverage to the skin while retaining a natural skin appearance. In particular, when this composition is applied onto darker skins, it has been found that coverage may be achieved whilst imparting minimal ashiness, or even whilst imparting no ashiness. Likewise, when this composition is applied to lighter skins (e.g. Caucasian skin), it has been found that coverage may be achieved whilst imparting minimal pastiness, or even whilst imparting no pastiness. More generally, when this composition is applied onto skin, it has been found that coverage may be achieved whilst imparting a minimal white/blue hue, or even whilst imparting no white/blue hue. It has been found also that, when applied onto the skin area surrounding eyes, skin discoloration may be corrected. Without wishing to be bound by any theory, it is believed that the incorporation of transparent

iron oxide particles in combination with the iron-containing titanium dioxide particles according to the present invention minimizes the reflectance of violet & blue light. More specifically, it is believed that the combination of these particles allows the minimization of the reflectance of violet & blue light firstly by limiting the proportion of violet & blue light being reflected but secondly also by absorbing, at least partially, some of the remaining violet & blue light that is reflected.

The reflectance of violet & blue light may be determined by measuring the coloration of the cosmetic composition at an incident light at 45° angle and by analyzing the reflectance spectrum. This coloration may be measured using the Spectraflash 600 spectrophotometer supplied by DataColor (DataColor International, UK), using a CMC (Colour measurement Committee) tolerancing method. CMC tolerancing method is closely matched to the visual acceptability response to the human eye.

Iron-containing titanium dioxide particles

The cosmetic composition according to the present invention comprises iron-containing titanium dioxide particles having an average surface area from 1 $m^2/g$ to 30 $m^2/g$, preferably from 1 $m^2/g$ to 20 $m^2/g$, more preferably from 1 $m^2/g$ to 15 $m^2/g$. The iron-containing titanium dioxide particles are pigmentary grade particles.

The iron-containing titanium dioxide particles comprise from 1% to 15%, preferably from 5% to 10%, iron by weight of titanium dioxide. If these particles comprise less than 1% iron by weight of titanium dioxide, then the combination of these particles with the transparent iron oxide particles may not significantly minimize the reflectance of violet & blue light. If these particles comprise more than 15% iron by weight of titanium dioxide, the combination of these particles with the transparent iron oxide particles may not minimize further the reflectance of violet & blue light and the iron-containing titanium dioxide particles may impart yellowness to the cosmetic composition and/or to the skin.

The iron-containing titanium dioxide particles may have an average primary particle size of at least 105 nm, preferably from 120nm to 500nm and, more preferably from 140 nm to 375 nm. The average primary particle size of these particles may be measured by transmission electron microscopy followed by particle size analysis or by laser diffraction. For example, the average primary particle size may be measured by particle size analysis using the apparatus Zeiss Particle Analyser TGZ-3 provided by Carl Zeiss Inc.

The iron-containing titanium dioxide may be surfaced-treated and/or coated, using conventional treatments. The surface treatment and/or the coating of metal oxide particles may be used to reduce the reactivity of the surface and also to provide specific properties to these particles, e.g. hydrophobic properties, hydrophilic particles, dispersibility, etc. Examples of treatment surface and/or coating are titanate, silane, amino silicone, methicone, dimethicone, silica, magnesium myristate, chitosan, lauroyl lysine, and lecithin. When the particle is coated, then the coated particle comprises the particles itself and an outer layer (coating). The particle may be coated with an inorganic compound and/or an organic compound using conventional methods.

The iron-containing titanium dioxide particles may further comprise an additional metallic element. This metallic element may be selected from aluminium, zinc, sodium, potassium, magnesium, phosphorus and the like.

Titanium dioxide may be selected from rutile, anastase or mixtures thereof. Preferably, titanium dioxide is rutile.

Preferably, the iron is distributed throughout the titanium dioxide particles. The use of these particles in combination with transparent iron oxide particles is particularly advantageous for minimizing the reflectance of violet & blue light. More preferably, these particles comprise a hydrophobic coating being substantially free of iron and the iron is distributed throughout the titanium dioxide particles. Alternatively, these particles comprise a hydrophilic coating being substantially free of iron and the iron is distributed throughout the titanium dioxide particles. As used herein, the term "substantially free" means that the coating comprises less than 1% iron, preferably less than 0.5% iron, more preferably no iron. Suitable raw materials are manufactured by Ishihara Sangyo Kaisha Ltd under the trade name FX50. This raw material comprises iron-containing titanium dioxide particles comprising the titanium dioxide and the iron, these particles being surface treated with aluminium hydroxide present as aluminium oxide $Al_2O_3$, and optionally, being coated hydrophobically with dimethicone and methicone. This raw material comprises 86% to 92% titanium dioxide by weight of the total particle, 1% to 3% $Al_2O_3$ by weight of the total particle, 5% to 10% iron by weight of titanium dioxide, and has an average surface area from 10 $m^2/g$ to 15 $m^2/g$ and an average primary particle size from 150 nm to 190 nm. This raw material may be manufactured according to the process detailed in the Japanese patent application H5-231041, filed on 24 August 1993, which is incorporated herein by reference.

Alternative suitable raw materials are manufactured by Nikko Rica Corporation under the trade name Fincera®.

The cosmetic composition may comprise from 0.05% to 20%, preferably from 1% to 15%, more preferably from 2% to 12.5%, still more preferably from 3% to 10%, iron-containing titanium dioxide particles by weight of the total composition. The proportion of these particles may vary depending on the desired level of coverage and/or shade of the product. For example, to minimise ashiness, when the composition is expected to be used onto darker skins for obtaining a high coverage, it is preferable that this composition comprises a high proportion of iron-containing titanium dioxide particles, for example from 5% to 10% particles by weight of the total composition.

Iron oxide particles

The cosmetic composition according to the present invention comprises iron oxide particles having an average surface area from 30 $m^2/g$ to 150 $m^2/g$, preferably from 50 $m^2/g$ to 150 $m^2/g$, more preferably from 60 $m^2/g$ to 150 $m^2/g$. These particles are transparent particles being conventionally used in cosmetic compositions. These particles are not pigmentary grade particles.

The iron oxide particles may have an average primary particle size of less than or equal to 100 nm. The average primary particle size of these particles may be measured by transmission electron microscopy.

The iron oxide particles may be selected from transparent yellow iron oxide particles, transparent red iron oxide particles, transparent black iron oxide particles or mixture thereof. Preferably, the iron oxide particles are selected from transparent yellow iron oxide particles, transparent red iron oxide particles or mixture thereof. Transparent yellow iron oxide is also known as goethite, ferric oxide hydrate or CI 77492. Transparent red iron oxide is also known as haematite, ferric oxide and CI 77491. Transparent black iron oxide is known as magnetite, ferrous-ferric oxide and CI 77499. Examples of commercially available transparent iron oxide particles include CM3F30TRR, CM3F40TRR, CM3F30TRY and CM3F40TRY supplied by Kobo; Trionix® materials from Noviant; and, the SunChroma® materials from Sun Chemicals.

The cosmetic composition may comprise from 0.05% to 10%, preferably from 0.1% to 5%, more preferably from 0.1% to 4%, iron oxide particles having an average surface area from 30 $m^2/g$ to 150 $m^2/g$ by weight of the total composition.

The cosmetic composition may comprise a weight ratio of iron-containing titanium dioxide particles : transparent iron oxide particles from 3:1 to 300:1, preferably from 4:1 to 150:1, more preferably from 5:1 to 70:1.

Cosmetically acceptable carrier

The cosmetic composition comprises a cosmetically acceptable carrier. This carrier may be any conventional cosmetically acceptable carrier known by the skilled person. This carrier may be an anhydrous carrier or an emulsion. Preferably, this carrier is an oil-in-water emulsion or a water-in-oil emulsion. More preferably, this carrier is a silicone-in-water emulsion or a water-in-silicone emulsion. Still more preferably, this carrier is a water-in-silicone emulsion.

Preferred water-in-silicone emulsions comprise a discontinuous aqueous phase and a continuous silicone phase. The water-in-silicone emulsion may comprise from 0.1% to 70%, preferably from 1% to 50%, more preferably from 5% to 40%, aqueous phase by weight of the total emulsion.

It is preferable that the iron oxide particles and the iron-containing titanium dioxide particles are incorporated into the continuous phase. For example, when the carrier is a water-in-silicone emulsion, it is preferred that the transparent iron oxide particles and the iron-containing titanium dioxide particles are incorporated into the continuous silicone phase.

The cosmetic composition may further comprise at least one hydrophobic organic UV-sunscreen being a cinnamic derivative. The inventors have surprisingly found that the composition according to the present invention, when further comprising at least one hydrophobic organic UV-sunscreen being a cinnamic derivative, this composition may provide coverage to the skin while retaining a natural skin appearance. In particular, when this composition is applied onto darker skins, it has been found that coverage may be achieved whilst imparting minimal ashiness, or even whilst imparting no ashiness. Likewise, when this composition is applied to lighter skins (e.g. Caucasian skin), it has been found that coverage may be achieved whilst imparting minimal pastiness, or even whilst imparting no pastiness. More generally, when this composition is applied onto skin, it has been found that coverage may be achieved whilst imparting minimal white/blue hue, or even whilst imparting no white/blue hue. Without wishing to be bound by any theory, it is believed that the incorporation of at least one hydrophobic organic UV-sunscreen being a cinnamic derivative, in combination with transparent iron oxide particles and iron-containing titanium dioxide particles according to the present invention, minimizes further the reflectance of violet & blue light.

Suitable examples of cinnamic derivative sunscreens may be found in the CTFA International Cosmetic Ingredient Dictionary and Handbook, 7th edition volume 2, pp.1672, edited by Wenning and Mc Ewen (The Cosmetic, Toiletry, and Fragrance Association, Inc., Washington, D.C. 1997).

Preferably, the cinnamic derivative may be selected from 2-ethylhexyl-p-methoxycinnamate, diethanolamine methoxycinnamate, 2-ethoxyethyl-p-methoxycinnamate, or mixture thereof. More preferably, the cinnamic derivative is 2-ethylhexyl-p-methoxycinnamate.

Example of commercially available ethylhexyl-p-methoxycinnamate includes Uvinul MC 80 from BASF.

The composition may comprise from 0.1% to 16%, preferably from 0.2% to 12%, more preferably from 0.5% to 10%, most preferably from 1% to 7.5%, of at least one hydrophobic organic UV-sunscreen being a cinnamic derivative, by weight of the total composition.

The cosmetic composition may further comprise a cross-linked organopolysiloxane elastomer. The cross-linked organopolysiloxane elastomer is selected from emulsifying cross-linked organopolysiloxane elastomer, non-emulsifying

cross-linked organopolysiloxane elastomer or mixtures thereof. As used herein, the term "non-emulsifying" when employed in relation to the cross-linked organopolysiloxane elastomer includes cross-linked organopolysiloxane elastomer comprising no polyoxyalkylene or polyglyceryl unit. As used herein, the term "emulsifying" when employed in relation to the cross-linked organopolysiloxane elastomer includes cross-linked organopolysiloxane elastomer comprising at least one polyoxyalkylene (e.g., polyoxyethylene or polyoxypropylene) or polygyceryl unit.

Compositions according to the invention may comprise from 0.01% to 15%, preferably from 1% to 12.5%, more preferably from 2% to 10%, cross-linked organopolysiloxane elastomer by weight of the total composition. If present, the composition may comprise from 0.01% to 15%, preferably from 0.01% to 1%, emulsifying cross-linked organopolysiloxane elastomer by weight of the total composition and from 0.01% to 15%, preferably from 2% to 10%, non-emulsifying cross-linked organopolysiloxane elastomer by weight of the total composition.

No specific restriction exists as to the type of curable organopolysiloxane composition that can serve as starting material for the cross-linked organopolysiloxane elastomer. Examples in this respect are addition reaction-curing organopolysiloxane compositions which cure under platinum metal catalysis by the addition reaction between SiH-containing diorganopolysiloxane and organopolysiloxane having silicon-bonded vinyl groups; condensation-curing organopolysiloxane compositions which cure in the presence of an organotin compound by a dehydrogenation reaction between hydroxyl-terminated diorganopolysiloxane and SiH-containing diorganopolysiloxane; condensation-curing organopolysiloxane compositions which cure in the presence of an organotin compound or a titanate ester, by a condensation reaction between an hydroxyl-terminated diorganopolysiloxane and a hydrolyzable organosilane; peroxide-curing organopolysiloxane compositions which thermally cure in the presence of an organoperoxide catalyst; and organopolysiloxane compositions which are cured by high-energy radiation, such as by gamma-rays, ultraviolet radiation, or electron beams.

Preferred non-emulsifying cross-linked organopolysiloxane elastomers are dimethicone/vinyl dimethicone crosspolymers. Examples of commercially available dimethicone/vinyl dimethicone crosspolymers include DC 9040, DC 9045 and DC 9041 from Dow Corning Corporation; SFE 839 from General Electric; KSG-15, KSG-16 and KSG-18 from Shin Etsu Chemical Company Ltd; and Gransil™ line of materials from Grant Industries. Examples of commercially available lauryl dimethicone/vinyl dimethicone crosspolymers include KSG-31, KSG-32, KSG-41, KSG-42, KSG-43, and KSG-44 from Shin Etsu Chemical Company Ltd.

Preferred emulsifying cross-linked organopolysiloxane elastomers are polyoxyalkylene-modified elastomers formed from divinyl compounds, particularly siloxane polymers with at least two free vinyl groups, reacting with Si-H linkages on a polysiloxane backbone. Examples of commercially available emulsifying cross-linked organopolysiloxane elastomers include KSG-21 and KSG-210 and KSG-320 from the Shin-Etsu Chemical Company Ltd. Examples of commercially available emulsifying cross-linked organopolysiloxane elastomers comprising polyglyceryl units include KSG 710 and KSG-800 from the Shin-Etsu Chemical Company Ltd.

The cosmetic composition may further comprise an oil. This oil may be selected from volatile oils, non-volatile oils or mixtures thereof. As used herein, the term "non-volatile" when employed in relation to an oil includes oils that fulfill at least one of the following definitions: (a) the oil exhibits a vapour pressure of no more than 0.2mm Hg at 25°C and one atmosphere pressure; (b) the oil has a boiling point at one atmosphere of at least 300°C. As used herein, the term "volatile" when employed in relation to oils includes materials that are not "non-volatile" as previously defined herein.

This composition may comprise from 1% to 80%, preferably from 10% to 70%, more preferably from 15% to 65%, oil by weight of the total composition. If present, the cosmetic composition may comprise from 0.1% to 20%, preferably from 1 to 10%, non-volatile oil by weight of the total composition.

Non-volatile oils may be selected from non-volatile silicone oils, non-volatile hydrocarbon oils and mixtures thereof. Suitable non-volatile silicone oils include linear polymethylsiloxanes and, preferably, non-volatile silicone oils are high molecular weight dimethicones. Examples of commercially available linear polymethylsiloxanes include DC 200 Fluid 20Cst, DC 200 Fluid 100Cst, DC 200 Fluid 350Cst from Dow Corning Corporation.

Suitable non-volatile hydrocarbon oils include branched esters of diglycerin or triglycerin or the esters or 1,2,3,4 butane triol or erythritol, di erythritol or tri erthyritol. Preferably, non-volatile hydrocarbon oils comprise erythrityl triethylhexanoate (available as Salacos E-38 from Nisshin Oilio) and Polyglyceryl-2 triisostearate (available as Cosmol 43V from Nisshin Oilio), diethyl hexyl carbonate (available as Tegosoft DEC from Degussa), dicapryl Ether (available as Cetiol OE from Cognis AG), dicapryl Carbonate (available as Cetiol CC from Cognis AG), isononyl isononanoate (available as Lanol 99 from Seppic), tridecyl Neopentanoate (supplied as Ceraphyl 55 from International Speciality Products), or mixture thereof.

Volatile oils may be selected from volatile silicone oils, both functionalised and non-functionalised, volatile hydrocarbon oils and mixtures thereof. Volatile oil useful in the present invention may exhibit one or more of the following characteristics - it may be saturated or unsaturated, have a straight or branched chain or a cyclic structure.

Examples of volatile hydrocarbons oils include polydecanes such as isododecane and isodecane (e.g., Permethyl-99A which is available from Presperse Inc.) and the $C_7$-$C_{15}$ isoparaffins (such as the Isopar Series available from Exxon Chemicals).

The volatile silicone oil may be selected from volatile cyclic silicone oils corresponding to the formula:

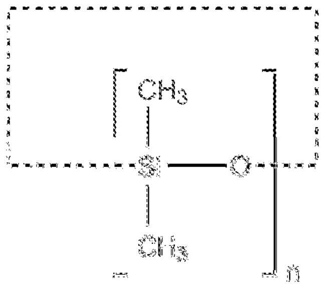

wherein n is from 3 to 7,
volatile linear silicone oils corresponding to the formula

$$(CH_3)_3Si\text{-}O\text{-}[Si(CH_3)_2\text{-}O]_m\text{-}Si(CH_3)_3$$

wherein m is from 1 to 20 preferably from 3 to 12, or mixture thereof.

Preferably, the cyclic volatile silicone oil is selected cyclopentasiloxane, cyclohexasiloxane or mixture thereof. Examples of commercially available volatile cyclic silicone oils include DC 244, DC 245, DC 344, and DC 345 from Dow Corning Corp.; SF-1204 and SF-1202 Silicone Fluids from Momentive Performance Materials; GE 7207 and 7158 from General Electric Co.); and, SWS-03314 from SWS Silicones Corp.

Preferably, the linear volatile silicone oil is a linear polymethylsiloxane. Example of commercially available linear polymethylsiloxanes include DC 200 Fluid, 5Cst from Dow Corning Corp.

The composition may further comprise an emulsifier. The emulsifier may be selected from nonionic emulsifiers, anionic emulsifiers, cationic emulsifiers, zwitterionic emulsifiers, amphoteric emulsifiers or mixtures thereof. Emulsifiers are known in the art. See, e.g., McCutcheon's, Detergents and Emulsifiers, North American Edition (1986), published by Allured Publishing Corporation.

When the cosmetically acceptable carrier is a water-in-silicone emulsion, emulsifiers are preferably selected from polyoxyalkylene copolymers, polyglyceryl copolymers or mixtures thereof. Polyoxyalkylene copolymers, also known as silicone polyethers, are described in detail in US 4,268,499. Example of commercially available polyoxyalkylene copolymers include DC5225C or DC2-5185C (PEG/PPG-18/18 dimethicone available as blend with cyclopentasiloxane) from Dow Corning Corp.; and, KF6017 or KF6028 (PEG-9 dimethicone) from Shin-Etsu Inc. Example of commercially available polyglyceryl emulsifiers include KF6100 and KF6104 from Shin-Etsu Inc.

The composition may comprise from 0.01% to 15%, more preferably from 0.1% to 10%, still more preferably from 1.0% to 5%, and most preferably from 1.0% to 3%, emulsifiers by weight of the total composition.

The cosmetic composition may further comprise at least one additional organic sunscreen active. As used herein, the expression "additional organic sunscreen active" means an organic sunscreen active other than a hydrophobic organic UV-sunscreen being a cinnamic derivative. The additional organic sunscreen active may be selected from hydrophilic organic sunscreen actives, hydrophobic organic sunscreen actives, or mixtures thereof. Suitable examples of sunscreens may be found in the CTFA International Cosmetic Ingredient Dictionary and Handbook, 7th edition volume 2, pp.1672, edited by Wenning and Mc Ewen (The Cosmetic, Toiletry, and Fragrance Association, Inc., Washington, D.C. 1997).

The additional hydrophobic organic sunscreen active may be selected from alkyl β,β-diphenylacrylate derivatives, α-cyano β,β-diphenylacrylate derivatives, anthranilate derivatives, benzophenone derivatives, camphor derivatives, dibenzoylmethane derivatives, p-aminobenzoic derivatives, salicylic derivatives, triazine derivatives, or mixtures thereof. More preferably, the hydrophobic organic sunscreen active is selected from 4-(1,1-dimethylethyl)-4'-methoxydibenzoylmethane; 4-isopropyldibenzoylmethane; 4-(1,1-dimethylethyl)-4'-methoxydibenzoylmethane, 2-ethylhexyl-2-cyano-3,3-diphenylacrylate, or mixture thereof.

Example of commercially available 4-(1, 1-dimethylethyl)-4'-methoxydibenzoylmethane, also known as butyl methoxydibenzoylmethane or Avobenzone, include Parsol TM 1789 from Givaudan Roure S. A. and Eusolex TM 9020 from Merck & Co., Inc. Example of commercially available 4-isoproplydibenzoylmethane, also known as isopropyldibenzoylmethane, include Eusolex TM 8020 from Merck & Co., Inc. Examples of commercially available 2-ethylhexyl-2-cyano-3,3-diphenylacrylate, also known as Octocrylene, include Uvinul N539 SG from BASF; and Eusolex OCR from Rona/Merck.

Preferably, the hydrophilic organic sunscreen active is 2-phenylbenzimidaole-5-sulfonic acid. Example of commercially available 2-phenylbenzimidaole-5-sulfonic acid, also known as PBSA, includes Eusolex 232 from Rona/Merck.

The composition may comprise from 0.1% to 16%, preferably from 0.2% to 12%, more preferably from 0.5%

to 10%, of at least one additional organic sunscreen active by weight of the total composition.

The cosmetic composition may further comprise particles having an average primary particle size from 0.001 μm to 100 μm, preferably from 0.001 μm to 75 μm. As used herein, the expression "spherical particles having an average primary particle size from 0.001 μm to 100 μm" means particles other than iron oxide particles having an average surface area from 30 $m^2/g$ to 150 $m^2/g$ and iron-containing titanium dioxide particles having an average surface area from 1 $m^2/g$ to 30 $m^2/g$ and comprising from 1% to 15% iron by weight of titanium dioxide. The primary average particle size of these particles can be measured by any conventional methods.

Commercially available particles include Tospearl 145A or Tospearl 2000 (methylsilsesquioxane resin microspheres) from Momentive Performance Materials; Micropearl M 100 (microspheres of polymethylmethacrylates) from Seppic; Trefil E 506C or Trefil E 505C (particles of crosslinked polydimethylsiloxanes) from Dow Corning Toray Silicone, Orgasol 2002D Nat C05 (particles of polyamide) from Atochem, Dynospheres (polystyerene microspheres) from Dyno Particles, FloBead (ethylene acrylate copolymer) sold by Kobo, Microthene (polyethylene), Micropoly 220 (polyethylene), silica, or mixtures thereof.

The composition may comprise from 0.01% to 40%, preferably from 1% to 30%, more preferably from 1% to 20%, particles having an average primary particle size from 0.001 μm to 100 μm by weight of the total composition.

The cosmetic composition may also comprise additional metal oxide particles. As used herein, the expression "additional metal oxide particles" means metal oxide particles other than iron oxide particles having an average surface area from 30 $m^2/g$ to 150 $m^2/g$ and iron-containing titanium dioxide particles having an average surface area from 1 $m^2/g$ to 30 $m^2/g$ and comprising from 1% to 15% iron by weight of titanium dioxide. The additional metal oxide particles may be selected from pigmentary grade iron oxide particles, sunscreen grade metal oxide particles, or mixtures thereof.

The pigmentary iron oxide particles have an average primary particle size greater than 100 nm, preferably greater than 100 nm to 500 nm. These particles may have an average surface area of from 1 $m^2/g$ to less than 30 $m^2/g$, preferably from 1 $m^2/g$ to 25 $m^2/g$, more preferably from 1 $m^2/g$ to 20 $m^2/g$. These particles may be selected from pigmentary yellow iron oxide particles, pigmentary red iron oxide particles, pigmentary black iron oxide particles or mixture thereof. Preferably, the iron oxide particles are selected from pigmentary yellow iron oxide particles, pigmentary red iron oxide particles or mixture thereof. Pigmentary yellow iron oxide is also known as goethite, ferric oxide hydrate or CI 77492. Pigmentary red iron oxide is also known as haematite, ferric oxide and CI 77491. Pigmentary black iron oxide is known as magnetite, ferrous-ferric oxide and CI 77499. These particles may be surface-treated and/or coated, using conventional treatments. Examples of commercially available pigmentary iron oxide particles include Cosmetic Red Iron Oxide C7054, Cosmetic Yellow Iron Oxide C7055, Unipure Black LC989 AS-EM from LCW-Sensient Cosmetic Technologies. The composition may comprise from 0.05% to 15%, preferably 0.1% to 12%, even more preferably 0.5% to 10%, pigmentary iron oxide particles by weight of the total composition.

The sunscreen metal oxide particles have an average primary particle size equal to or less than 100 nm, preferably from 5 nm to 80 nm, more preferably from 10 nm to 75 nm. These particles may have an average surface area of more than 30 $m^2/g$, preferably from 40 $m^2/g$ to 150 $m^2/g$. These particles may be selected from sunscreen grade titanium dioxide, sunscreen grade zinc oxide or mixtures thereof, preferably sunscreen grade titanium dioxide particles. These particles may be surface treated and/or coated, using conventional treatments. Examples of commercially available sunscreen metal oxide particles include M262 from Kemira Corp., TTO S-3 and TTO S-4 from Ishihara Corp. The composition may comprise from 0.05% to 15%, preferably 0.5% to 10%, more preferably from 1% to 5%, sunscreen grade metal oxide particles by weight of the total composition.

Advantageously, the cosmetic composition comprises from 1% to 20%, preferably 2.5% to 17.5%, more preferably from 5% to 15%, total metal oxide particles by weight of the total composition. As used herein, the expression "total metal oxide particles" means the iron oxide particles having an average surface area from 30 $m^2/g$ to 150 $m^2/g$, the iron-containing titanium dioxide particles having an average surface area from 1 $m^2/g$ to 30 $m^2/g$ and comprising from 1% to 15% iron by weight of titanium dioxide and, if present, the additional metal oxide particles.

The cosmetic composition may further comprise a skin-conditioning agent. The skin-conditioning agent may be selected from humectants, exfoliants, emollients or mixtures thereof. Humectants includes polyhydric alcohols. Preferably, polyhydric alcohols may be selected from glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerine or mixtures thereof. More preferably, polyhydric alcohols is glycerine. The composition may comprise from 0.01% to 30%, preferably from 1% to 20%, more preferably from 1% to 15%, skin-conditioning agent by weight of the total composition.

A variety of additional optional ingredients may be incorporated into the compositions of the present invention. Non-limiting examples of these additional ingredients include additional skin care actives such as peptides (e.g., Matrixyl [pentapetide derivative]), farnesol, bisabolol, phytantriol, urea, guanidine (e.g., amino guanidine); vitamins and derivatives thereof such ascorbic acid, vitamin A (e.g., retinoid derivatives such as retinyl palmitate or retinyl proprionate), vitamin E (e.g., tocopherol acetate), vitamin B3 (e.g., niacinamide) and vitamin B5 (e.g., panthenol) and the like and mixtures thereof; hexaminidine compounds, salts or derivatives thereof; sugar amine; self-tanning agent (e.g. dehydroxyacetone);

structuring agent; hydrophilic gelling agents; anti-acne medicaments (resorcinol, salicylic acid, and the like; antioxidants (e.g., phytosterols, lipoic acid); flavonoids (e.g., isoflavones, phytoestrogens); skin soothing and healing agents such as aloe vera extract, allantoin and the like; chelators and sequestrants; and agents suitable for aesthetic purposes such as essential oils, fragrances, skin sensates, opacifiers, aromatic compounds (e.g., clove oil, menthol, camphor, eucalyptus oil, and eugenol).

According to the second aspect of the present invention, the cosmetic composition is preferably used as a color cosmetic composition. More preferably, the cosmetic composition is used as a foundation. It is advantageous to provide a foundation comprising iron oxide particles having an average surface area from 30 $m^2/g$ to 150 $m^2/g$, iron-containing titanium dioxide particles having an average surface area from 1 $m^2/g$ to 30 $m^2/g$ and comprising from 1% to 15% iron by weight of titanium dioxide and, a cosmetically acceptable carrier in order to cover and/or to mask skin irregularities and/or skin imperfections and/or skin tonal variations without imparting pastiness or ashiness. It is also advantageous to provide a foundation further comprising at least one hydrophobic sunscreen active being a cinnamic derivative, preferably 2-ethylhexyl-p-methoxycinnamate. It is preferably applied from 0.25 $mg/cm^2$ to 3 $mg/cm^2$ of cosmetic composition onto skin.

According to the third aspect of the present invention, the cosmetic composition is preferably used as a composition to correct skin discoloration surrounding the eye. The target area for treatment is typically the skin covering the orbit (*i.e.*, the bony cavity of the skull that contains the eye). This discoloration is commonly referred to as "dark circles around the eyes". Often this discoloration is perceived to have a blue-color, particularly in the skin under the eyes. As described above, the present cosmetic compositions provide coverage to darker skin without imparting "ashiness" (*i.e.*, a white/blue hue). The present cosmetic compositions can be selectively applied to the skin surrounding the eyes so as to provide coverage while minimizing the blue hue typically perceived with such eye discoloration. In one embodiment, the cosmetic composition is applied to the skin surrounding the eyes.

Methodology

The reflectance of the violet & blue light from a composition according to the present invention - versus control and/or comparative composition(s) - may be measured by using an integrating sphere spectrophotometer and evaluating the reflectance curve of the composition at an incident light at 45° angle, i.e. by measuring the reflectance of the composition *in vitro*.

When comparing the composition according to the invention versus control and/or comparative compositions, it is preferable to conduct standardization between the different compositions (e.g. CMC (2,1) delta E value, %w/w and contrast ratio) before measuring the reflectance, analyzing and comparing the data.

CMC (2,1) delta E value

The coloration of the composition(s) may be measured using an integrating sphere spectrophotometer (e.g. Spectraflash 600 spectrophotometer supplied by DataColor International, UK) and using a CMC (Colour measurement Committee) tolerancing method - e.g. the CMC (2,1) equation (see British Standard BS:6923). The light source may be intended to represent average daylight and to have a correlated temperature of approximately 6500K (recognized as a standard by ISO 10526:1999) - e.g. a D65 illuminant at a 10° standard observer with the measurements being made using the specular inclusive mode. The composition may be applied onto a region of a hiding power chart (e.g. chart ref:301/2A supplied by Sheen Instruments Ltd, UK) using a Bird applicator (e.g. supplied by Paul N. Gardner Company Inc., USA) with a 0.012"(12 mils) cut depth, equating to a wet film thickness of about 0.006" (6 mils). The capture area is in the middle of the black and white regions of the card.

If the readings of the reflectance is taken immediately after application of the composition, then it is preferable the readings for the CMC (2,1) delta to be taken also immediately after application of the composition, i.e. when the composition is wet. If the readings of the reflectance is taken 24h after application of the composition, then it is preferable the readings for the CMC (2,1) delta to be taken also 24h after application of the composition, i.e. when the composition is dried.

The compositions to be tested are preferably comparable to each other in shade with a maximum CMC (2,1) delta E (see equation below) of 1.6 when wet or CMC (2,1) delta E = 0.6 after 24 hours of drying. L*, C* and h° refer to co-ordinates in 3D color space, where L* indicates how light to dark the color is, C* is a relative measure of the intensity of the hue (h°) of a surface, and h° indicates the hue angle of color.

The delta E values are calculated using the below equation:

$$dE_{CMC} = \sqrt{\left(\frac{dL^*}{lS_L}\right)^2 + \left(\frac{dC^*}{cS_C}\right)^2 + \left(\frac{dH^*}{S_H}\right)^2}$$

Wherein the terms 1 and c represent two constants whose value depends on the type of assessment made. When the CMC (2,1) value is required, 1=2 and c=1.

When the composition comprising transparent iron oxide particles, iron-containing titanium dioxide particles, and a cosmetically acceptable carrier is concerned, readings are taken immediately after application of the composition. When the composition comprising transparent iron oxide particles, iron-containing titanium dioxide particles, one hydrophobic sunscreen agent being a cinnamic derivative, and a cosmetically acceptable carrier is concerned, readings are taken 24 hours after application of the composition.

%w/w of materials

The compositions to be tested preferably include the same %w/w of materials (i.e. actives) of interest.

Contrast ratio

The compositions to be tested preferably have similar contrast ratios - i.e. with the maximum difference in contrast ratio between the compositions being 0.1 when measuring the composition wet, preferably 0.05 when measuring the composition dry. Contrast ratio is preferably calculated by measuring the tristimulus values using an integrating sphere spectrophotometer (as above) and calculating the ratio of the Y tristimulus value over black and white card. The light source is a D65 illuminant at a 10° standard observer. To calculate this contrast ratio, the composition may be applied onto a region of a hiding power chart (as above) using a Bird applicator (as above) with a 0.002" (2 mils) cut depth, equating to a wet film thickness of about 0.001" (1 mils). The composition is allowed to dry for 24 hours and readings are taken over the black card and then over the white card.

Measurement of the reflectance, analyzing and comparing the data

The reflectance may be measured on the whole composition in *vitro* over the black area of a coated hiding power chart (see above). The composition may be applied to a region of the card using a Bird applicator (as above) with a 0.012" (12 mils) cut depth, equating to a wet film thickness of about 0.006" (6 mils). When the composition comprising transparent iron oxide particles, iron-containing titanium dioxide particles, and a cosmetically acceptable carrier is concerned, the readings are taken immediately after application of the composition. When the composition comprising transparent iron oxide particles, iron-containing titanium dioxide particles, one hydrophobic sunscreen agent being a cinnamic derivative, and a cosmetically acceptable carrier is concerned, readings are taken 24 hours after application of the composition. Readings are taken using an integrating sphere spectrophotometer (as above). The light source is a D65 illuminant at a 10° standard observer and readings are made using the specular inclusive mode.

Given the inherent variability of *in vitro* data using thin film thickness, each composition should be drawn onto three separate cards (three reps).

The spectral curve for each composition is preferably plotted and the data is preferably examined in the blue region ranging from 400 nm and 500 nm and, more preferably, in the violet & blue region ranging from 420 nm and 450 nm.

Examples

The following examples further describe and demonstrate the preferred embodiments within the scope of the present invention. The examples are given solely for the purpose of illustration, and are not to be construed as limitations of the present invention since many variations thereof as possible without departing from its scope.

All weights provided in these examples are weights of the commercially available materials, including active (s) and/or solvent and/or by-products.

Examples 1 to 5

A liquid foundation of the present invention is prepared as follows: in a suitable vessel, water, glycerine, disodium EDTA and benzyl alcohol are added and mixed using conventional technology until a clear water phase is achieved. When the water phase is clear, the methylparabens are added and mixed again until clear. The resultant phase is mixed

with a Silverson SL2T or similar equipment on high speed (8,000 rpm, standard head). In a separate vessel, the dimethicone & dimethicone copolyol cross-polymer, cyclopentasiloxane, pigment dispersion, other oils, dispersant and the parabens are added and the mixture is milled using a Silverson SL2T on a high speed setting until a homogeneous mixture is created.

Following this step, the water phase and the silicone phase are combined and milled using the Silverson SL2T on a high speed setting until the water is fully incorporated and an emulsion is formed. The dimethicone crosspolymer & cyclopentasiloxane is then added and the mixture is mixed again using the Silverson on a high speed setting to generate the final product.

| Example | 1 | 2 | 3 | 4 | 5 |
|---|---|---|---|---|---|
| Ingredient (%w/w) | 100 | 100 | 100 | 100 | 100 |
| Dimethicone crosspolymer & Cyclopentasiloxane [1] | 25.0 | 20.0 | | | 30.0 |
| Dimethicone / Vinyl Dimethicone crosspolymer & Cyclopentasiloxane [2] | | | 20.0 | 50.0 | |
| Dimethicone & Dimethicone copolyol cross-polymer [3] | | 5.0 | 10.0 | | 0.5 |
| Cyclopentasiloxane [4] | 10.0 | 5.0 | 3.0 | 28.0 | 10. |
| PEG/PPG18/18 Dimethicone & Cyclomethicone [5] | 1.8 | 2.0 | | | 2.2 |
| Octyl Methoxy cinnamate | 4.0 | | | | |
| Octocrylene | | | | 4.0 | |
| Diethylhexyl carbonate [6] | 6.0 | | | | 2.0 |
| Dicapryl ether [7] | | 4.0 | | 1.9 | |
| Dicapryl Carbonate [8] | | | 2.0 | | |
| Sunscreen grade Titanium Dioxide & Cyclopentasiloxane[9] | 4.0 | | | 5.98 | 6.0 |
| Iron-containing Titanium Dioxide[10] | 8.5 | 6.0 | 8.85 | 8.0 | 5.0 |
| Pigmentary grade Titanium dioxide [11] | | 3.0 | | | 4.0 |
| Black Iron Oxide Pigment Dispersion[12] | 1.2 | 0.18 | 3.4 | 0.12 | 0.12 |
| Yellow Iron Oxide Pigment Dispersion [12] | | | 0.3 | 0.6 | |
| Red Iron Oxide Pigment Dispersion [12] | 1.8 | 0.55 | 3.6 | 0.4 | |
| Transparent Red Iron Oxide Dispersion [13] | 0.9 | 0.2 | 2.1 | 1.0 | 3.0 |
| Transparent Yellow Iron Oxide Dispersion [14] | | 0.1 | | | 0.3 |
| Propylparabens | 0.1 | 0.1 | 0.1 | | 0.1 |
| Ethylparabens | 0.1 | 0.1 | 0.1 | | 0.2 |
| Methylparabens | 0.1 | 0.1 | 0.1 | | 0.1 |
| Disodium EDTA | 0.1 | 0.1 | 0.1 | | 0.01 |
| Benzyl alcohol | 0.5 | 0.5 | 0.5 | | 0.25 |
| Sodium chloride | 2.0 | 2.0 | 2.0 | | |
| Glycerin | 10.0 | 12.0 | | | 7.0 |
| Niacinamide | 2.0 | 5.0 | 5.0 | | 0.5 |

(continued)

| Example | 1 | 2 | 3 | 4 | 5 |
|---|---|---|---|---|---|
| Water | qs | qs | qs | Nil | qs |
| 1: Supplied as Dow Corning 9040 by Dow Corning Corp - 2: Supplied as KSG15 by Shin-Etsu chemical company limited - 3: Supplied as KSG21 by Shin-Etsu chemical company limited - 4: Supplied as Dow Corning 245 by Dow Corning Corp - 5: Supplied as Dow Corning 2-5185C by Dow Corning Corp - 6: Supplied as Tegosoft DEC by Degussa - 7: Supplied as Cetiol OE by Degussa - 8: Supplied as Cetiol CC by Degussa - 9: Supplied as SAS-TTO-S3 by Miyoshi Kasei (35.3% solids) - 10: Supplied as FX50-DMC4 by Kobo - 11: Supplied as Titanium Dioxide (9729) treated with methicone (2%) by Sensient - 12: Supplied as Iron Oxide dispersions in cyclopentasiloxane by Kobo (CM3FA65EBH (65% solids), CM3FA70ERH (70% solids), CM3FA55EYH (53% solids)) - 13: Supplied as CM3F30TRR by Kobo (30% solids) - 14: Supplied as CM3F30TRY by Kobo (30% solids) | | | | | |

Example 6 to 10

A liquid foundation of the present invention is prepared as follows: in a suitable vessel, water, propylene glycol, methyl paraben, sodium chloride and poly vinyl pyrrolidone are added, heated and mixed using conventional technology until a clear water phase is achieved. The resultant phase is cooled to room temperature. In a separate vessel, cyclopentasiloxane, pigment dispersions, other oils, dispersant, propyl paraben, iron-containing titanium dioxide, transparent iron oxides and the waxes are added and the mixture is mixed whilst it is heated. Once the waxes are well dispersed, the mixture is milled using a Silverson SL2T on a high speed setting and allowed to cool to 55°C when the powders, trihydroxystearin and linear silicones are added. Once both phases are at room temperature, the water phase and the silicone phase are combined and milled using the Silverson SL2T on a high speed setting until the water is fully incorporated and an emulsion is formed.

| Examples | 6 | 7 | 8 | 9 | 10 |
|---|---|---|---|---|---|
| Ingredient (%w/w) | 100 | 100 | 100 | 100 | 100 |
| Cyclopentasiloxane [1] | 7 | 9 | 7 | 8 | 9 |
| Trihydroxystearin [2] | 0.1 | 1 | 0.1 | 1 | 1 |
| Polyglyceryl-4 Isostearate [3] | 0.2 | 0.2 | 0.2 | 0.2 | 0.2 |
| Propyl Paraben | 0.25 | 0.25 | 0.25 | 0.25 | 0.25 |
| PEG/PPG-18/18 Dimethicone & Cyclomethicone [4] | 23.75 | 20.5 | 23.75 | 19.8 | 20.5 |
| Lauryl Alcohol Ethoxylates [5] | 0.35 | 0.35 | 0.35 | 0.35 | 0.35 |
| Sunscreen Grade Titanium Dioxide [6] | 0.4 | 0.4 | 0.4 | 0.4 | 0.4 |
| Arachidyl Behenate | 0.6 | 0.6 | 0.6 | 0.6 | 0.6 |
| Silica | 0.25 | | 0.25 | | |
| Talc | 2.08 | 2.08 | 2.08 | 2.08 | 2.08 |
| Iron Containing Titanium Dioxide (FX50-DMC4) [7] | 8.5 | 6.0 | 8.85 | 8.0 | 5.0 |
| Titanium Dioxide (PPS 200-500nm) [8] | | 3.0 | | | 4.0 |
| Black Iron Oxide Pigment Dispersion [9] | 0.27 | 0.27 | 0.32 | 0.4 | 0.11 |
| Yellow Iron Oxide Pigment Dispersion [9] | | | | | 0.15 |
| Red Iron Oxide Pigment Dispersion [9] | 0.5 | 0.5 | | 0.5 | |
| Transparent Red Iron Oxide Dispersion [10] | 0.9 | 0.2 | 2.1 | 1.0 | 3.0 |
| Transparent Yellow Iron Oxide Dispersion [11] | | 0.1 | | | 0.3 |
| Talc - Ethylene / Methacrylate Copolymer [12] | 0.35 | 0.35 | | 0.35 | 0.5 |
| Aluminium Starch Octenylsuccinate [13] | 1 | 2 | 1 | 3 | 1 |
| Dimethicone 350cs [14] | 3 | | 3 | 2 | 1.5 |

(continued)

| Examples | 6 | 7 | 8 | 9 | 10 |
|---|---|---|---|---|---|
| Dimethicone 100cs [15] | 2 | 2 | 2 | | 2 |
| Deionised Water | qs | qs | qs | qs | qs |
| Methyl Paraben | 0.1 | 0.1 | 0.1 | 0.1 | 0.1 |
| Sodium Chloride | 1.5 | 2 | 1.5 | 1.9 | 0.8 |
| Propylene Glycol | 5 | 8 | 8 | 4 | 7 |
| Niacinamide | 1 | 1 | | 1 | |
| Poly vinyl pyrrolidone [16] | 0.5 | 0.5 | 0.5 | 0.5 | 0.5 |

1: Supplied as Dow Corning 245 by Dow Corning Corp - 2: Supplied as Thixcin from S.Black - 3: Supplied as Abil WE09 from Degussa - 4: Supplied as Dow Corning 5225C by Dow Corning Corp - 5: Supplied as Laureth 7 from Rhodia - 6: Supplied as MT100T from Tayca Corporation - 7: Supplied as FX50-DMC4 by Kobo - 8: Supplied as Titanium Dioxide (9729) treated with methicone (2%) by Sensient - 9: Supplied as Iron Oxide dispersions in cyclopentasiloxane by Kobo (CM3FA65EBH (65% solids), CM3FA70ERH (70% solids), CM3FA55EYH (53% solids)) - 10: Supplied as CM3F30TRR by Kobo (30% solids) - 11: Supplied as CM3F30TRY by Kobo (30% solids) - 12: Supplied as SPCAT -I2B by Kobo - 13: Supplied as Tospearl 200 by Momentive Chemicals - 14: Supplied as Dow Corning 200, 350Cs by Dow Corning Corp - 15: Supplied as Dow Corning 200, 100Cs by Dow Corning Corp - 16: Supplied as Luviskol K17 by BASF

Example 11

An eye contour treating composition of the present invention is prepared according to a conventional method for preparing cosmetic composition.

| Example | 11 |
|---|---|
| Ingredient (%w/w) | 100 |
| Cyclopentasiloxane [1] | 40 |
| Silica [2] | 0.20 |
| Iron Containing Titanium Dioxide (FX50-DMC4) [3] | 2.00 |
| Iron oxide CI 77491 [4] | 0.60 |
| Titanium dioxide [5] | 5.00 |
| Iron oxide CI 77499 [6] | 0.20 |
| Cyclopentasiloxane and Dimethicone Crosspolymer [7] | qs |
| Cyclopentasiloxane [8] | 0.40 |
| Polyethylene [9] | 3.00 |
| Polyethylene [10] | 3.00 |
| Ethyl panthenol | 0.50 |
| Vitamin E acetate | 0.50 |
| Palmitoyl pentapeptide-3 [11] | 0.50 |

(continued)

| | |
|---|---|
| Preservatives | 0.80 |
| 1: Supplied as SF-1202 Silicone Fluid by Momentive Performance Materials Inc. - 2: Supplied as Aerosil 300 from Evonik Degussa GmbH - 3: Supplied as FX50-DMC4 by Kobo - 4: Supplied as FAF40TRR by Kobo products Inc. - 5: Supplied as PM1P75CSI from Kobo products Inc. - 6: Supplied as FA60EBSI from Kobo Products Inc. - 7: Supplied as DC9045 by Dow Corning Corp. - 8: Supplied as D-5 - 9: Supplied as Microthene FN-510 from Equistar Chemicals - 10: Supplied as Micro Poly 220 from Micro Powders Inc. - 11: Supplied as Promatrixyl by Sederma | |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. Cosmetic composition comprising:

   (a) iron oxide particles having an average surface area from 30 $m^2/g$ to 150 $m^2/g$;
   (b) iron-containing titanium dioxide particles having an average surface area from 1 $m^2/g$ to 30 $m^2/g$ and comprising from 1% to 15% iron by weight of the titanium dioxide; and,
   (c) a cosmetically acceptable carrier.

2. Composition, according to claim 1, wherein the iron-containing titanium dioxide particles have an average surface area of from 1 $m^2/g$ to 20 $m^2/g$, preferably from 1 $m^2/g$ to 15 $m^2/g$.

3. Composition, according to claim 1 or 2, wherein the iron-containing titanium dioxide particles comprise from 5% to 10% iron by weight of the titanium dioxide.

4. Composition, according to any of the preceding claims, wherein the iron-containing titanium dioxide particles have an average primary particle size of at least 105nm, preferably from 120nm to 500nm, more preferably from 140nm to 375nm.

5. Composition, according to any of the preceding claims, comprising from 0.05% to 20%, preferably from 1% to 15%, more preferably from 2% to 12%, still more preferably from 3% to 10%, iron-containing titanium oxide particles by weight of the total composition.

6. Composition, according to any of the preceding claims, wherein the iron-containing titanium dioxide particles comprise a coating being substantially free of iron and wherein the iron is distributed throughout the titanium dioxide particles.

7. Composition, according to any of the preceding claims, wherein the iron oxide particles have an average surface area of from 50 $m^2/g$ to 150 $m^2/g$, preferably from 60 $m^2/g$ to 150 $m^2/g$.

8. Composition, according to any of the preceding claims, wherein the iron oxide particles have an average primary particle size of less than or equal to 100 nm.

9. Composition, according to any of the preceding claims, comprising from 0.05% to 10%, preferably from 0.1% to 5%, more preferably from 0.1% to 4%, iron oxide particles having an average surface area from 30 $m^2/g$ to 150 $m^2/g$ by weight of the total composition.

**10.** Composition, according to any of the preceding claims, wherein the cosmetically acceptable carrier is an emulsion, preferably a water-in-oil or an oil-in-water emulsion more preferably a water-in-silicone emulsion.

**11.** Composition, according to any of the preceding claims, comprising at least one hydrophobic organic sunscreen active being a cinnamic derivative.

**12.** Composition, according to the claim 11, comprising 2-ethylhexyl-p-methoxycinnamate.

**13.** Composition, according to any of the preceding claims, comprising a cross-linked organopolysiloxane elastomer.

**14.** Use of the compositions defined in claims 1 to 13 as a cosmetic skin foundation.

**15.** Use of the compositions defined in claims 1 to 13 to correct skin discoloration surrounding the eye.

Europäisches Patentamt
European Patent Office
Office européen des brevets

# EUROPEAN SEARCH REPORT

Application Number
EP 08 15 9449

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JP 2008 088317 A (KOSE CORP) 17 April 2008 (2008-04-17) * example 4 * | 1,14,15 | INV. A61K8/29 A61Q1/00 |
| A | DATABASE WPI Week 198718 Thomson Scientific, London, GB; AN 1987-125518 XP002510294 & JP 62 067014 A (SUNSTAR KK) 26 March 1987 (1987-03-26) * abstract * | 1,14,15 | |
| Y,D | DATABASE WPI Week 199519 Thomson Scientific, London, GB; AN 1995-144501 XP002510295 & JP 07 069636 A (ISHIHARA SANGYO KAISHA LTD) 14 March 1995 (1995-03-14) * abstract * | 1,14,15 | |
| A | US 5 837 050 A (OKUDA HARUO [JP] ET AL) 17 November 1998 (1998-11-17) * claims; examples 1,2 * | 1,14,15 | TECHNICAL FIELDS SEARCHED (IPC) A61K A61Q |

The present search report has been drawn up for all claims

| Place of search | Date of completion of the search | Examiner |
|---|---|---|
| Berlin | 14 January 2009 | Boeker, Ruth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 08 15 9449

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2009

| Patent document cited in search report | Publication date | Patent family member(s) | Publication date |
|---|---|---|---|
| JP 2008088317 A | 17-04-2008 | NONE | |
| JP 62067014 A | 26-03-1987 | JP 6021057 B | 23-03-1994 |
| JP 7069636 A | 14-03-1995 | JP 2852482 B2 | 03-02-1999 |
| US 5837050 A | 17-11-1998 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H5231041 B
- US 4268499 A


### Non-patent literature cited in the description


- CTFA International Cosmetic Ingredient Dictionary and Handbook. The Cosmetic, Toiletry, and Fragrance Association, Inc, 1997, vol. 2, 1672